(19) Europäisches Patentamt
European Patent Office
Office européen des brevets

(11) Veröffentlichungsnummer: **0 295 488 A2**

(12) # EUROPÄISCHE PATENTANMELDUNG

(21) Anmeldenummer: **88108644.1**

(51) Int. Cl.⁴: **C07D 221/14**

(22) Anmeldetag: **31.05.88**

(30) Priorität: **13.06.87 DE 3719850**

(43) Veröffentlichungstag der Anmeldung: **21.12.88 Patentblatt 88/51**

(84) Benannte Vertragsstaaten: **CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Schütze, Detlef-Ingo, Dr.**
**Roggendorfstrasse 51**
**D-5000 Köln 80(DE)**
Erfinder: **Wunderlich, Klaus, Dr.**
**Carl-Rumpff-Strasse 21**
**D-5090 Leverkusen(DE)**
Erfinder: **Reinhardt, Karl-Heinz**
**Carl-von-Ossietzky-Strasse 10**
**D-5090 Leverkusen(DE)**
Erfinder: **Wienkenhöver, Martin, Dr.**
**Gustav-Freytag-Strasse 2**
**D-5090 Leverkusen(DE)**

(54) **Verfahren zur Herstellung von Naphthalin-1,8-dicarbonsäureimiden.**

(57) Verfahren zur Herstellung von gegebenenfalls im Naphthalinring substituiertem Naphthalin-1,8-dicarbonsäureimid, dadurch gekennzeichnet, daß man gegebenenfalls im Naphthalinring substituiertes Naphthalin-1,8-dicarbonsäureanhydrid mit einem Ammoniumsalz im wäßrigen Medium bei einem pH-Wert in Nähe des Neutralpunktes umsetzt, danach auf einen pH-Wert von 1 bis 4 ansäuert und anschließend erneut einen pH-Wert in der Nähe des Neutralpunktes einstellt.

EP 0 295 488 A2

Xerox Copy Centre

## Verfahren zur Herstellung von Naphthalin-1,8-dicarbonsäureimiden

Die Erfindung betrifft ein Verfahren zur Herstellung von gegebenenfalls im Naphthalinring substituiertem Naphthalin-1,8-dicarbonsäureimid.

Naphthalin-1,8-dicarbonsäureimide sind wichtige Farbstoffzwischenprodukte. Insbesondere stellt das unsubstituierte Naphthalin-1,8-dicarbonsäureimid ein wertvolles Zwischenprodukt zur Herstellung von Perylentetracarbonsäurediimid dar, das wiederum durch Verseifung zum Perylentetracarbonsäuredianhydrid oder direkt die Basis zur Synthese der Perylentetracarbonsäure-Pigmente darstellt.

Ein seit längerer Zeit bekanntes Verfahren zur Herstellung von Naphthalin-1,8-dicarbonsäureimid und seinen Derivaten geht von Naphthalin-1,8-dicarbonsäureanhydrid aus, das in wäßrigem Ammoniak umgesetzt wird. Hierbei ist ein großer Ammoniaküberschuß nötig, um hohe Ausbeuten zu erreichen (s. z.B. Chem. Zentralblatt 1959, S. 2432: Referat von J.allg. Chem. (russ.) 28 (90), 692-695, März 1958). Außerdem ist es erforderlich, daß das eingesetzte Naphthalin-1,8-dicarbonsäureanhydrid frei von Naphthalsäure ist, da diese in ihr Diammoniumsalz überführt wird, das nur schwer weiter reagiert.

In der DE-OS 2 137 242 ist ein weiteres Verfahren beschrieben, wobei festes Naphthalin-1,8-dicarbonsäureanhydrid mit gasförmigem Ammoniak bei Temperaturen von 120 °C bis 130 °C unter Druck umgesetzt wird. Dieses Verfahren ist aber nur mit einem hohen technischen Aufwand durchzuführen.

Das erfindungsgemäße Verfahren zur Herstellung von gegebenenfalls im Naphthalinring substituiertem Naphthalin-1,8-dicarbonsäureimid ist dadurch gekennzeichnet, daß man gegebenenfalls im Naphthalinring substituiertes Naphthalin-1,8-dicarbonsäureanhydrid mit einem Ammoniumsalz im wäßrigen Medium bei einem pH-Wert in Nähe des Neutralpunktes, vorzugsweise einem pH-Wert von 6,5 bis 7,5, umsetzt, danach auf einen pH-Wert von 1 bis 5, vorzugsweise 2,5 bis 3,5, ansäuert und anschließend erneut einen pH-Wert in der Nähe des Neutralpunktes, vorzugsweise 6,5 bis 7,5, einstellt.

Überraschenderweise ist die Qualität des eingesetzten Naphthalin-1,8-dicarbonsäureanhydrids nicht entscheidend für die Qualität und Ausbeute an Imid.

Insbesondere dient das Verfahren zur Herstellung von Naphthalin-1,8-dicarbonsäureimiden der Formel

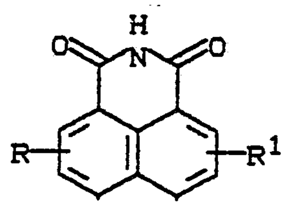

(I)

in der

R, $R^1$ Wasserstoff, Alkyl, vorzugsweise $C_1$-$C_4$-Alkyl, z.B. Methyl, Ethyl, Alkoxy, vorzugsweise $C_1$-$C_4$-Alkoxy, z.B. Methoxy, Ethoxy, Halogen, vorzugsweise Chlor, Brom, N,N-Dialkylamino, vorzugsweise N,N-Di-$C_1$-$C_4$-alkylamino, z.B. N,N-Dimethylamino, N,N-Diethylamino und Nitro bezeichnen.

Zur Herstellung der Verbindungen der Formel I geht man von den entsprechend substituierten Naphthalin-1,8-dicarbonsäureanhydriden bzw. vom unsubstituierten Anhydrid aus.

Diese Naphthalin-1,8-dicarbonsäureanhydride sind literaturbekannt oder können in Analogie zu Literaturbekannten Verfahren hergestellt werden.

Besonders bevorzugt dient das neue Verfahren zur Herstellung von unsubstituiertem Naphthalin-1,8-dicarbonsäureimid.

Als Ammoniumsalze finden Ammoniumsalze anorganischer und organischer Säuren, z.B. Ammoniumchlorid, -acetat, -hydrogensulfat, -nitrat, -carbonat und insbesondere Ammoniumsulfat Verwendung.

Pro Mol Anhydrid werden vorzugsweise 1 bis 1,5 Äquivalente Ammoniumsalz eingesetzt. Besonders bevorzugt setzt man pro Mol Anhydrid 0,52 bis 0,75 Mol, ganz besonders bevorzugt 0,55 bis 0,6 Mol $(NH_4)_2SO_4$ ein. Dies entspricht einem Überschuß an Ammoniak von etwa 10 bis 20 %, während nach der oben zitierten Vorschrift aus Chemisches Zentralblatt ein Ammoniaküberschuß von 200 % erforderlich ist.

Das erfindungsgemäße Verfahren wird vorzugsweise im Temperaturbereich von 10 °C bis zum Siedepunkt des Reaktionsgemischs, besonders bevorzugt bei 70 bis 100 °C, durchgeführt.

Zur Einstellung des wäßrigen Reaktionsgemischs aus Anhydrid und Ammoniumsalz auf einen pH-Wert in der Nähe des Neutralpunktes werden üblicherweise ein Alkali- oder Erdalkalimetallhydroxid oder ein tertiäres Amin, z.B. Triethylamin, in Form wäßriger Lösungen oder Anschlämmungen verwendet. Bevorzugt

erfolgt die pH-Einstellung mit Natronlauge.

Zur Einstellung des pH-Wertes von 1 bis 4 dient insbesondere eine Mineralsäure, vorzugsweise Schwefelsäure.

Zur Vervollständigung der Reaktion empfiehlt es sich, beim pH-Wert in der Nähe des Neutralpunktes 0,5 bis 3 Stunden, bevorzugt 1 bis 1,5 Stunden, nachzurühren.

Eine Ausgestaltung des erfindungsgemäßen Verfahrens besteht darin, das Ansäuern auf pH 1 bis 4 und die Einstellung des pH-Wertes auf die Nähe des Neutralpunktes mehrfach, vorzugsweise ein-, zwei- oder dreimal zu wiederholen. Dies empfiehlt sich insbesondere dann, wenn das eingesetzte Anhydrid von minderer Qualität, z.B. durch Naphthalsäure verunreinigt, ist.

Die Aufarbeitung des Reaktionsgemischs erfolgt in üblicher Weise, z.B. durch Absaugen, Neutralwaschen und Trocknen.

Nach dem erfindungsgemäßen Verfahren erhält man Naphthalin-1,8-dicarbonsäureimide mit sehr hohe Qualitäten und sehr hohen Ausbeuten. Die folgenden Beispiele dienen der Veranschaulichung der Erfindung; Teile bedeuten Gewichtsteile und Prozente Gewichtsprozente.

Beispiel 1

148,7 Teile Naphthalsäureanyhdrid (97,6 %ig + 2,0 % Naphthalin-1,8-dicarbonsäure) und 59,4 Teile Ammoniumsulfat werden in 750 Teilen Wasser eingetragen und in ca. 30 Minuten auf 90 °C erhitzt. Es stellt sich ein pH-Wert von 3,0 bis 3,5 ein. Nun werden innerhalb von 1 Stunde ca. 75 Teile 50 %ige Natronlauge möglichst gleichmäßig zugetropft, so daß der pH-Wert auf 6,5 bis 7,5 ansteigt. 1 Stunde wird bei 90 °C nachgerührt.

Anschließend wird der pH-Wert durch Zutropfen von ca. 27 Teilen 78 %iger Schwefelsäure auf 3,0 gestellt und 30 Minuten bei 90 °C gerührt.

Dann werden in 30 Minuten weitere 40 Teile 50 %ige Natronlauge zugetropft, um den pH-Wert erneut auf 6,5 bis 7,5 einzustellen.

Nachdem 2 Stunden bei 90 °C nachgerührt worden ist, wird auf 60 °C abgekühlt, abgesaugt, mit Wasser neutral gewaschen und bei 100 °C getrocknet.

Es werden 144,9 Teile Naphthalin-1,8-dicarbonsäureimid mit einem Gehalt von 99,3 % erhalten. Dies entspricht einer Reinausbeute von 97,8 % der Theorie.

Beispiel 2

148,7 Teile Naphthalsäureanhydrid (96,2 %ig + 0,9 % Naphthalin-1,8-dicarbonsäure) werden analog Beispiel 1 umgesetzt. Dabei wird aber ein zweites Mal mit Schwefelsäure auf einen pH-Wert von 3,0 eingestellt und anschließend mit 50 %iger Natronlauge auf pH 6,5 bis 7,5.

Man erhält 142,9 Teile Naphthalin-1,8-dicarbonsäureimid mit einem Gehalt von 98,7 %, was einer Reinausbeite von 98 % der Theorie entspricht.

Beispiel 3

Man verfährt analog zu Beispiel 1, wobei 148,7 Teile Naphthalsäureanhydrid (90,4 %ig + 1,5 % Naphthalin-1,8-dicarbonsäure) eingesetzt werden. Es wird jedoch insgesamt 3 mal ein pH-Wert von 3,0 und anschließend von 6,5 bis 7,5 eingestellt.

Es werden 136,4 Teile Naphthalin-1,8-dicarbonsäureimid mit einem Gehalt von 98,2 % erhalten. Dies entspricht einer Reinausbeute von 98,2 % der Theorie.

## Ansprüche

1. Verfahren zur Herstellung von gegebenenfalls im Naphthalinring substituiertem Naphthalin-1,8-dicarbonsäureimid, dadurch gekennzeichnet, daß man gegebenenfalls im Naphthalinring substituiertes Naphthalin-1,8-dicarbonsäureanhydrid mit einem Ammoniumsalz im wäßrigen Medium bei einem pH-Wert in Nähe des Neutralpunktes umsetzt, danach auf einen pH-Wert von 1 bis 4 ansäuert und anschließend erneut einen pH-Wert in der Nähe des Neutralpunktes einstellt.

2. Verfahren gemäß Anspruch 1 zur Herstellung von Naphthalin-1,8-dicarbonsäureimiden der Formel

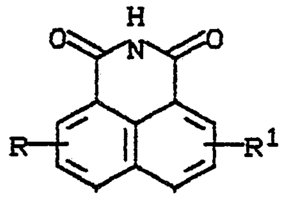

(I)

in der

R, $R^1$ Wasserstoff, Alkyl, Alkoxy, Halogen, N,N-Dialkylamino und Nitro bezeichnen.

3. Verfahren gemäß Anspruch 1 zur Herstellung von Naphthalin-1,8-dicarbonsäureimid.

4. Verfahren gemäß den Ansprüchen 1 bis 3, dadurch gekennzeichnet, daß man mit Ammoniumsulfat umsetzt.

5. Verfahren gemäß den Ansprüchen 1 bis 4, dadurch gekennzeichnet, daß man pro Mol Anhydrid 1 bis 1,5 Äquivalente Ammoniumsalz einsetzt.

6. Verfahren gemäß den Ansprüchen 1 bis 5, dadurch gekennzeichnet, daß man in den entsprechenden Schritten bei einem pH-Wert von 6,5 bis 7,5 arbeitet.

7. Verfahren gemäß den Ansprüchen 1 bis 6, dadurch gekennzeichnet, daß man auf einen pH-Wert von 2,5 bis 3,5 ansäuert.

8. Verfahren gemäß den Ansprüchen 1 bis 7, dadurch gekennzeichnet, daß man das Ansäuern auf pH 1 bis 4 und die Einstellung des pH-Wertes auf die Nähe des Neutralpunktes mehrfach wiederholt.